Europäisches Patentamt

**European Patent Office**

Office européen des brevets

(19)

(11) Publication number : **0 385 610 B1**

(12) # EUROPEAN PATENT SPECIFICATION

(45) Date of publication of patent specification :
**16.03.94 Bulletin 94/11**

(21) Application number : **90301589.9**

(22) Date of filing : **14.02.90**

(51) Int. Cl.$^5$ : **A61K 39/295,** A61K 39/29,
A61K 39/12, A61K 39/13,
C12N 15/62, C12N 15/36,
C12N 15/43

(54) Conjugates.

(30) Priority : **14.02.89 GB 8903313**

(43) Date of publication of application :
**05.09.90 Bulletin 90/36**

(45) Publication of the grant of the patent :
**16.03.94 Bulletin 94/11**

(84) Designated Contracting States :
**BE CH DE DK ES FR GB IT LI NL SE**

(56) References cited :
**EP-A- 0 174 759**
**EP-A- 0 271 302**
**EP-A- 0 287 395**
**SCIENCE, vol. 233, 25th July 1986, pages
472-475; F. DELPEYROUX et al.: "A poliovirus
neutralization epitope expressed on hybrid
hepatitis B surface antigen particles"**
**NATURE, vol. 330, no. 6146, 26th November -
2nd December 1987, pages 381-384, London,
GB; B.E. CLARKE et al.: "Improved im-
munogenicity of a peptide epitope after fusion
to hepatitis B core protein"**

(56) References cited :
**METHODS IN ENZYMOLOGY, vol. 178, 1989,
pages 659-676, Academic Press, Inc.; M.J.
FRANCIS et al.: "[42] peptide vaccines based
on enhanced immunogenicity of peptide
epitopes presented with T-cell determinants
or hepatitis B core protein"**

(73) Proprietor : **THE WELLCOME FOUNDATION
LIMITED
Unicorn House 160 Euston Road
London NW1 2BP (GB)**

(72) Inventor : **Rowlands, David John
The Wellcome Foundation Limited, Langley
Court
Beckenham, Kent BR3 3BS (GB)**
Inventor : **Clarke, Berwyn Ewart
The Wellcome Foundation Limited, Langley
Court
Beckenham, Kent BR3 3BS (GB)**
Inventor : **Francis, Michael James
The Wellcome Foundation Limited, Langley
Court
Beckenham, Kent BR3 3BS (GB)**

(74) Representative : **Stott, Michael John et al
The Wellcome Foundation Limited Group
Patents & Agreements Langley Court
Beckenham Kent BR3 3BS (GB)**

EP 0 385 610 B1

## Description

This invention relates to conjugates which comprise polypeptides incorporating an antigenic determinant coupled to a carrier, to their preparation and to their use in raising antibodies.

The aims of a good vaccine should be to provide a rapid onset of immunity that is of long duration and provides immunological memory for a subsequent inoculation or encounter with the infectious agent. The vaccine formulation must also be easy to administer, stable, have minimal side effects and produce broad protection in the recipient. These aims are largely met by many existing commercial products. However, conventional vaccines based on inactivated infectious agents do present problems. These include the undefined nature of the immunizing antigen, whether the product is truely innocuous, risk associated with handling large amounts of infectious material, stability and limitations on the mode of presentation, generally resulting from problems of stability.

In an attempt to produce more stable and defined vaccines scientists have been studying the immune response to many infectious agents in detail in order to identify the critical epitopes involved in providing protective immunity. Armed with this knowledge it is now possible to mimic such epitopes by producing short peptides and to use these as the basis of a vaccine. The advantages of such peptide based vaccines are numerous. They are chemically defined, stable indefinitely and no infectious material is involved in their manufacture. Furthermore, they can be designed to stimulate the appropriate immune response and provide the opportunity for using novel delivery systems and for targetting the antigen. From the manufacturers viewpoint they should also reduce the need for a large scale production plant and for complex downstream processing of the product.

Despite these clear advantages a number of criticisms have been levelled at peptide-based vaccines. These include the requirements for undefined carrier proteins and the belief that the immunogenicity of a peptide antigen could never approach that of the native organism. It was generally assumed that due to their relatively small molecular size many synthetic peptides would behave like haptens and would require coupling to a large "foreign" protein carrier to enhance their immunogenicity. Immunization with such conjugates often resulted in the production of anti-peptide antibodies that totally failed to recognise the native protein or infectious agent due to the method of peptide/carrier linkage. Other problems, of particular relevance to vaccination, that could be encountered were hypersensitivity to the "foreign" carrier protein and poor batch to batch reproducibility of the conjugates.

There has recently been interest in the use of hepatitis B core antigen (HBcAg) as a carrier protein. Clarke et al, Nature 330, 381-384, 1987 describe a fusion protein composed of the major antigenic site of the VP1 capsid protein of foot-and-mouth disease virus (FMDV) fused to the amino-terminus of HBcAg. The fusion protein self-assembled into regular 27 nm core-like particles. These particles were almost as immunogenic with respect to FMDV as the native virus itself.

EP-A-0271302 claims immunogenic polypeptide conjugates comprising HBcAg operatively linked through an amino acid residue side chain to a polypeptide immunogen. It also claims immunogenic fusion proteins comprising HBcAg protein operatively linked by a peptide bond to a pathogen related immunogen. Specific T cell stimulating polypeptides are claimed too.

We have investigated chemically coupling polypeptides to HBcAg particles. Each time, however, satisfactory results could not be obtained. In particular:

1. We sought to couple hepatitis B surface antigen (HBsAg) to HBcAg via side chain amino groups on HBcAg by derivatising the HBcAg particles with succinimidyl-4-(N-maleimido-methyl)-cyclohexane-1-carboxylate (SMCC). Little or no derivatisation of the HBcAg particles occurred.

2. The approach in 1. was repeated but using m-maleimido-benzoyl-N-hydroxysuccinimide ester (MBS) instead of SMCC. Again, little or no derivatisation of the HBcAg particles occurred.

3. Coupling of HBsAg to HBcAg particles was tried with glutaraldehyde. Coupling occurred at a high concentration of glutaraldehyde. However, the glutaraldehyde badly damaged the HBsAg. There was a severely reduced response to HBsAg when the resulting complexes were administered to mice.

4. We sought to couple a Cys-containing FMDV VP1 141-160 peptide to side chain amino groups on HBcAg using bis(maleimide)methyl ester (BMME). The HBcAg particles were reduced, derivatised with BMME and reacted with the peptide. Although coupling occurred, only low antibody responses to FMDV were induced by the resulting material.

In order to overcome these difficulties, we expressed a fusion protein composed of a short Lys-containing sequence fused to the amino terminus of HBcAg. We then coupled peptides to this modified HBcAg protein via the side chain amino group of the Lys residue. Coupling proceeded successfully. The resulting conjugates raised both antipeptide and neutralising antibody. The method is simple, efficient and can allow the peptide to be coupled in a defined operation.

Accordingly, the present invention provides particles which are composed of HBcAg provided with a N-terminal extension incorporating a Lys residue within the first fourteen N-terminal amino acid residues of the extension and to which are coupled via the side chain amino group of the said Lys residue a polypeptide which presents an antigenic epitope.

Any desired polypeptide may be coupled to the modified HBcAg particles. However, present on the polypeptide there needs to be a group such as $-NH_2$ or -SH capable of being coupled to the side chain amino group of the Lys residue in the N-terminal extension of HBcAg. The polypeptide typically presents a foreign epitope, i.e. an epitope which is not an epitope of HBcAg.

The polypeptide may comprise an antigenic epitope capable of raising neutralising antibody, for example an epitope of an infectious agent such as a virus, bacterium or protozoan. It may be an epitope of a non-infectious agent such as a growth hormone fragment. The polypeptide may comprise repeats of an epitope, for example up to eight or up to four copies of an epitope. Two copies of an epitope may therefore be present in the polypeptide. A polypeptide may comprise two or more different epitopes, for example three or four.

As examples of viruses whose epitopes may be presented there may be mentioned hepatitis A virus, hepatitis B virus, influenza virus, foot-and-mouth disease virus, poliovirus, herpes simplex virus, rabies virus, human immunodeficiency virus type 1 (HIV-1), HIV-2, simian immunodeficiency virus (SIV), human rhinovirus (HRV), dengue virus and yellow fever virus. The polypeptide coupled to the modified HBcAg may be therefore HBsAg or a polypeptide comprising the major FMDV VP1 antigenic site. A protozoan whose epitopes may be provided is the malaria parasite <u>Plasmodium</u> <u>falciparum</u>.

A peptide incorporating the major FMDV VP1 antigenic site which may be employed comprises a sequence starting at a residue from 137 to 142 and ending at a residue from 160 to 162 of VP1 of FMDV, for example of any of the FMDV serotypes. Typical sequences are VP1 residues 140 to 162, 140 to 160, 137 to 162, 137 to 160 or 141 to 160. A polypeptide comprising tandem repeats of such a sequence or mixed serotype tandem repeats of such a sequence can be coupled to the modified HBcAg particles.

Suitable peptides comprising tandem repeats of the major FMDV VP1 antigenic site are peptides comprising consecutive sequences, each sequence made up of no more than 30 amino acid residues and comprising the same immunogenic sequence. This immunogenic sequence is:

(i) a sequence starting at a residue of from 137 to 145 and ending at a residue from 150 to 162 of VP1 of FMDV sub- type $0_1$, or

(ii) a sequence of corresponding amino acid residues of another subtype of serotype 0 or of a subtype of a different FMDV serotype.

The peptides may therefore be tandem repeats of an immunogenic sequence. Alternatively, a peptide composed of consecutive sequences may be provided in which one or more of the sequences comprises additional amino acid residues not actually part of the repeated immunogenic sequence. Such additional amino acid residues may be present to link each immunogenic sequence. Such a linking sequence may be composed of up to six amino acid residues, for example from 1 to 3. Without such linking residues, each immunogenic sequence is bonded directly to the next.

The peptides can comprise any number of repeats of the immunogenic sequence. For example, from two to eight and, more preferably, from two to four repeats may be present. The peptides may or may not end at the C-terminal and/or N-terminal with a non-natural cysteine residue.

The sequence comprising the immunogenic FMDV sequence consists of no more than 30 amino acid residues. The length of the sequence depends on the length of the immunogenic sequence although additional linking amino acid residues and possibly a C-terminal and/or N-terminal non-natural cysteine may be present too. Preferably, however, each consecutive sequence is no more than 26 residues long.

The peptides present repeats of the major FMDV VP1 immunogenic site. The major FMDV epitope is typically defined by at least amino acid residues 142 to 160 of the VP1 capsid protein. This applies in particular to serotype $O_1$. An immunogenic sequence which may therefore be repeated is that defined by VP1 amino acid residues 142 to 160 of FMDV serotype $O_1$, optionally extending down to amino acid 137 at the N-terminal and/or up to amino acid 162 at the C-terminal, or by corresponding amino acids of another serotype. Typical sequences are VP1 residues 140 to 162, 140 to 160, 137 to 162 or 137 to 160 of serotype O or A, for example of subtypes $0_1$ and $A_{12}$. These typical sequences may be repeated twice, for example. Useful peptides are, using the one-letter code:

```
(137-162,O₁)-(137-162,O₁)-Cys:

(NRNAVPNLRGDLQVLAQKVARTLPTS)ₓ₂C;

(137-162,O₁)-(137-162,O₁)-Gly:

(NRNAVPNLRGDLQVLAQKVARTLPTS)ₓ₂G;  and

(137-162,A₁₂)-(137-162,A₁₂)-Cys:

(YSASGSGVRGDLGSLAPRVARQLPAS)ₓ₂C.
```

Smaller immunogenic sequences of the FMDV epitope may be presented, however. For example, the sequence defined by VP1 residues 145 to 150 of serotype $O_1$ may be presented in this way. Consequently, the FMDV sequence which can be repeated may be defined more broadly by VP1 residues 145 to 150 of serotype $O_1$, optionally extending down to amino acid 137 at the N-terminal and/or up to amino acid 162 at the C-terminal, or by corresponding amino acids of another serotype. Useful smaller peptides are:

```
(145-150,O₁)-(145-160,O₁)-Cys:

RGDLQVRGDLQVLAQKVARTLPC;  and

(145-150,O₁)-(145-150,O₁)-(145-160,O₁)-Cys:

RGDLQVRGDLQVRGDLQVLAQKVARTLPC.
```

Suitable peptides comprising mixed serotype tandem repeats of the major FMDV VP1 antigenic site are represented by the formula (I):

$$C' - X - Y - Z - C''  \quad (I)$$

in which X represents (i) a sequence starting at a residue from 137 to 142 and ending at a residue from 160-162 of VP1 of FMDV subtype $0_1$ or (ii) a sequence of corresponding amino acid residues of another subtype of serotype 0 or of a subtype of a different FMDV serotype;

Y represents a direct bond or a linking sequence of up to six amino acid residues;

Z represents a sequence defined as for X but of a different serotype than that of the sequence denoted by X; and

C' and C" each independently represent an optional cysteine residue.

Each immunogenic sequence X and Z is defined by VP1 amino acid residues 142 to 160 of FMDV serotype $O_1$, optionally extending down to amino acid 137 at the N-terminal and/or up to amino acid 162 at the C-terminal, or by corresponding amino acids of another subtype of serotype 0 or of a subtype of a different FMDV serotype. Typical sequences are VP1 residues 140 to 162, 140 to 160, 137 to 162 or 137 to 160.

The immunogenic sequences X and Z are of different serotypes. There are seven FMDV serotypes: O, A, C, Asia 1, SAT 1, SAT 2 and SAT 3. A useful peptide comprises $O_1$ and $A_{12}$ sequences in either order. The immunogenic sequences may be linked directly or via up to six, for example from 1 to 3, amino acid residues. Preferably, a C-terminal non-natural cysteine residue is present. Preferred peptides are, according to the one-letter code:

```
(137-162,O₁)-(137-162,A₁₂)-Cys:



NRNAVPNLRGDLQVLAQKVARTLPTS-YSASGSGVRGDLGSLAPRVARQLPAS-C;  and

(137-162,A₁₂)-(137-162,O₁)-Cys:

YSASGSGVRGDLGSLAPRVARQLPAS-NRNAVPNLRGDLQVLAQKVARTLPTS-C.
```

A preferred polypeptide for coupling to the modified HBcAg particles comprises the HRV NIm-II epitope of EP-A-0287395. This epitope is defined by amino acid residues 156 to 164 of VP2 of HRV2 or equivalent amino acid residues of another HRV. These amino acids may be replaced by other amino acids which do not affect the antigenicity of the sequence. For HRV2, the NIm-II sequence is

<p style="text-align:center;">VKAETRLNP.</p>

Equivalent amino acid residues of other HRV's are the VP2 amino acid residues corresponding to the VP2 amino acid residues 156 to 164 of HRV2. In other words, they can be the counterpart VP2 residues of another HRV serotype. These can readily be determined by lining up the VP2 sequence of another HRV with the VP2 sequence of HRV2. This is a straightforward matter because of the homology between VP2 sequences of different types of HRV.

The polypeptides may be relatively short peptides of up to 50, for example of up to 40 or of up to 30, amino acid residues. Alternatively they may be longer of up to, for example 100 or 200 amino acid residues in length. They may be proteins. They may have been obtained by chemical synthesis or by recombinant DNA methodologies.

The particles to which the polypeptides are coupled consist essentially of HBcAg provided with a N-terminal extension which incorporates a Lys residue. HBcAg self-assembles into particles 27 nm in diameter. The modified HBcAg's also self-assemble to form core-like particles. More than one Lys residue may be present in the N-terminal extension. Up to six Lys residues, for example up to four Lys residues, may be provided.

The N-terminal extension may have any appropriate length provided the HBcAg with the extension can self-assemble into core-like particles and provided a Lys residue in the extension is exposed available for coupling. The extension may be up to 250, for example up to 200 or up to 100 amino acid residues long. A short extension may be up to 60, for example up to 40, 20, 10 or 5, amino acid residues long.

A suitable extension comprises residues 95 to 102, for example 95 to 104, of the VP1 capsid protein of a strain of poliovirus type 1 (PV1) such as the Sabin or Mahoney strain:

| | | | | | |
|---------|-----|-----|--------|---|-----------|
| Sabin | PV1 | VP1 | 95-102 | : | SASTKNKD |
| Sabin | PV1 | VP1 | 95-104 | : | SASTKNKDKL |
| Mahoney | PV1 | VP1 | 95-102 | : | PASTTNKD |
| Mahoney | PV1 | VP1 | 95-104 | : | PASTTNKDKL |

This shows the sequences according to the one-letter code (Eur. J. Biochem. 138, 9-37, 1984) in which K denotes Lys. These sequences span the proposed major antigenic site on PV1 VP1. Another suitable extension comprises residues 156 to 164, for example 156 to 170, of the VP2 capsid protein of HRV2 or equivalent amino acid residues of another HRV. For HRV2, these residues are:

**156-164: VKAETRLNP**

**156-170: VKAETRLNPDLQPTE**

The PV1- or HRV- derived sequences may be provided with linker sequences at either or each end, for example of up to 5 or of up to 3 amino acid residues. For any N-terminal extension of HBcAg, there is at least one Lys residue within the first fourteen N-terminal residues, for example one Lys residue within the first five or first twelve such residues or two Lys residues within the first fourteen N-terminal residues. For a selected polypeptide it is wished to couple, it may be appropriate to choose a HBcAg with a N-terminal extension comprising a Lys residue close to the N-terminus of the extension. Also, the N-terminal extension may desirably be hydrophilic.

A modified HBcAg provided with a N-terminal extension comprising one of these sequences or any other sequence incorporating a Lys residue may be obtained by genetic engineering, for example according to JP-A-196299/88. More specifically, such a modified HBcAg may be obtained by providing a gene encoding the modified HBcAg in an appropriate expression vector and expressing the modified HBcAg in a host transformed with the vector.

A gene encoding the modified HBcAg may be prepared by synthesising a DNA sequence encoding the desired N-terminal extension and, with appropriate linkers as necessary, ligating it to the 5'-end of a DNA sequence encoding HBcAg. The resulting DNA sequence can thus be provided in an expression vector under the control of appropriate transcriptional and translational regulatory sequences. The modified HBcAg is expressed in a suitable procaryotic or eucaryotic host such as a strain of E. coli, Salmonella or yeast and recovered as particles.

An expression vector, such as a plasmid, capable of expressing HBcAg may be employed where there is an appropriate restriction site at the 5'-end of the DNA sequence encoding HBcAg. A DNA sequence encoding the desired N-terminal extension for HBcAg is inserted at that site. A fusion protein with the N-terminal extension linked to the amino-terminus of HBcAg is expressed.

A suitable expression vector is plasmid pBc404. E. coli (JM 101) harbouring this plasmid was deposited at the National Collection of Industrial and Marine Bacteria, Aberdeen, GB on 9 February 1989 under accession number NCIB 40111. This plasmid codes for an ampicillinase gene as selection marker and possesses a strong bacterial promoter, tac, located upstream of the HBcAg gene. The presence of restriction sites EcoRI and Bam-HI allows the insertion of a synthetic oligonucleotide coding for any desired N-terminal extension for HBcAg.

Conjugates are prepared according to the invention by coupling the polypeptide presenting an antigenic determinant to the modified HBcAg via the side chain amino group of the Lys residue present within the final fourteen N-terminal amino acid residues of the modified HBcAg. This is typically achieved by reacting the modified HBcAg with a bifunctional reagent capable of linking to an amino group and to a sulphydryl group. The thus-derivatised modified HBcAg is reacted with the polypeptide which, if not possessing a free sulphydryl group, has been modified so that it does possess such a group.

Suitable bifunctional reagents include SMCC, MBS and N-succinimidyl-3-(2-pyridyldithio)proprionate (SPDP). The bifunctional reagents generally include a group capable of forming a peptide link and a group capable of forming a disulphide or thioether link. A sulphydryl group may be provided on the polypeptide it is wished to couple to the modified HBcAg by the provision of a N-terminal and/or C-terminal Cys residue or by reaction of amino functions with 2-iminothiolane or the N-hydroxysuccinimide ester of 3-(3-dithiopyridyl)propionate or S-acetylthioglycolic acid. After reaction with S-acetylthioglycolic acid N-hydroxysuccinimide ester (SATA) deacetylation of the SATA with, for example, hydroxylamine produces free -SH groups.

In practice, the modified HBcAg particles are generally provided in a buffer such as phosphate buffer at a pH of about 7. A molar excess of the bifunctional agent in an inert solvent, for example an aprotic organic solvent such as dimethylformamide, is added. Derivatisation of the HBcAg particles occurs. Excess of the bifunctional agent is removed, by filtration for example. Excess of the polypeptide to be coupled to the HBcAg particles is then added. Coupling occurs and the resulting conjugates composed of the polypeptide linked to the modified HBcAg particles are recovered, for example by filtration.

The modified HBcAg core particles may be carboxymethylated before use. The purpose of this is to block sulphydryl groups on the core particles prior to derivatisation and therefore to prevent core proteins from cross-linking when a bifunctional reagent is used to couple the N-terminal side chain amino group on the core particles to a -SH group on a polypeptide. Carboxymethylation can be achieved by reacting the core particles with iodoacetamide. Excess reagent can be separated by gel filtration or dialysis.

The conjugates are useful for raising antibody to the antigenic determinant presented by the polypeptide linked to the HBcAg particles. The conjugates therefore may be used as vaccines for humans or animals. Vaccination is achieved by administering to a human or animal an effective amount of the conjugate. An oral route or a parenteral route such as sub-cutaneously, intravenously or intramuscularly may be adopted. Typically, the conjugate is administered in an amount of 1 to 1,000 μg per dose, more preferably 10 to 100 μg per dose, by either the oral or the parenteral route.

For administration, a conjugate is typically formulated with a pharmaceutically acceptable carrier or diluent. Conventional formulations, carriers, adjuvants and diluents may be employed. These will of course be determined by the route of administration. Suitable carriers and diluents include Freund's incomplete adjuvant (IFA), aluminium hydroxide, saponin, DEAE-dextran, muramyl dipeptide, mineral oils, neutral oils such as miglycol, vegetable oils such as arachis oil, "Iscoms", liposomes, Pluronic (trade mark) polyols or the Ribi adjuvant system (GB-A-2189141).

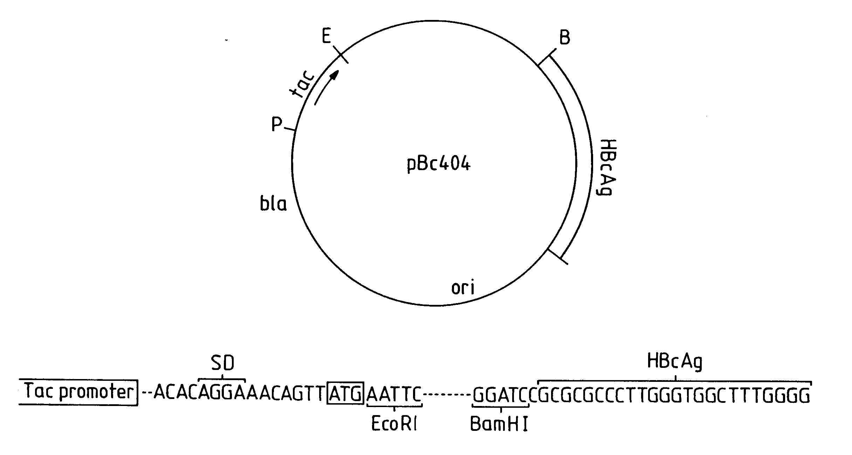

The following Examples illustrate the invention. In the accompanying Figure plasmid pBc404 is shown. B, E and P denote restriction sites for BamHI, EcoRI and PstI respectively; tac denotes the tac promoter; ori denotes the origin of replication; bla denotes β-lactamase and SD denotes the Shine-Dalgarno sequence.

Example 1: Preparation of HBcAg provided at its N-terminus with a short extension comprising PV1 Mahoney VP1 residues 95 to 104 (PV core)

An expression plasmid for PV core was prepared, based on the parent plasmid pBc404 shown in the Figure. Synthetic oligonucleonucleotides representing amino acids 95 to 104 of VP1 from PV1 Mahoney were ligated into pBc404 using T4 ligase by standard procedures. The synthetic oligonucleotides, how they anneal together and the coding sequence of the N-terminal extension are as follows:

1. AATTCAGATAATCCGGCTAGTACTACCAACAAAGÀTAAG (39)

2. GATCCTTATCTTTGTTGGTAGTACTAGCCGGATTATCTG (39)

AATTCAG ATAATCCAGC TAGTACTACC AACAAAGATA AG
   GTC TATTAGGTCG ATCATGATGG TTGTTTCTAT TCCTAGG

```
          10            20         30         40
ATGAATTCAGATAATCCAGCTAGTACTACCAACAAAGATAAGGATCC.....core
   M   N   S   D   N   P   A   S   T   T   N   K   D   K  D
   |_____|  |_____|

   LINKER                   POLIOVIRUS
```

Bacteria (E. coli JM 101) harbouring the recombinant plasmid were induced for expression using 60 µg/ml of isopropyl-β-D-thiogalactopyranoside (IPTG) for 6 hours. Cells were harvested by centrifugation, lysed by lysozyme and non-ionic detergent treatment. Bacterial debris was removed by centrifugation at 10000 rpm for 5 minutes.

Supernatant samples were fractionated on 15-45% linear sucrose gradient at 50000 rpm for 1 hour 15 minutes at 20°C. Particles were detected by optical density at 260 nm. The particles were concentrated by centrifugation at 40000 rpm for 1 hour or used directly for derivatisation after dialysis of sucrose.

Example 2: Coupling of the peptide composed of FMDV VP1 residues 141-160, which has also a C-terminal Cys residue, (FMDV 141-160 Cys) to PV cores

PV cores, purified on two consecutive sucrose gradients, were passed at a concentration of 5 mg/ml in 10 mM phosphate buffer of pH 7.2 through a Sephadex (trade mark) G100 column. The PV cores in 10 mM phosphate buffer at a concentration of 2 mg/ml were then derivatised by adding 1/20 volume of SMCC dissolved in dry dimethylformamide so that the final concentration of SMCC in the PV core sample is a 50 times molar excess relative to the PV core protein.

After 30 minutes at room temperature, the SMCC was removed by filtration through Sephadex G100 in 10 mM phosphate buffer, pH 7.2. Freshly dissolved FMDV 141-160 Cys was added in 1/10 volume to give a 10 times molar excess of the peptide with respect to the derivatised PV cores. After stirring at room temperature for 2 hours, uncoupled peptide was separated from the derivatised PV cores by filtration through Sephadex G200. The PV cores with the peptide attached were recovered.

Example 3: Coupling of HRV2 peptide to PV cores

Following the procedure described in Example 2, the HRV2 peptide which encompasses the NIm-II epitope and which has the sequence:

VKAETRLNPDLQPTC

was coupled to PV cores. The PV cores with peptide attached were recovered.

7

Example 4: Carboxymethylation of PV cores

PV cores after dialysis of sucrose and concentration by centrifugation were reacted in an amount of 200 μg/ml with 10mM iodoacetamide in 0.5M Tris, pH 8.0, for 1 hour at room temperature in the dark. Excess reagent was separated from the thus carboxymethylated PV cores by gel filtration.

Example 5: Coupling of hepatitis B surface antigen (HBsAg) to carboxymethylated PV cores

Carboxymethylated PV cores from Example 4 were derivatised with an equimolar amount of SMCC in dimethylformamide (DMF). The amount of SMCC was one-twentieth the volume of DMF. HBsAg particles expressed in yeast were derivatised at 200 μg/ml with S-acetylthioglycolic acid N-hydroxysuccinimide ester (SATA), again at equimolar ratios in DMF. The amount of SATA was also one-twentieth the volume of DMF. After deacetylation of the SATA with hydroxylamine to produce free -SH groups, equal volumes of the two derivatised particles, each of 200 μg/ml, were mixed. The PV cores with HBsAg attached were recovered.

Example 6: Coupling of FMDV peptide and HRV2 peptide to carboxymethylated PV cores

The FMDV 141-160 Cys peptide was coupled to carboxymethylated PV cores in accordance with Example 5. In a separate experiment, the HRV2 peptide described in Example 3 was coupled in the same way to carboxymethylated PV cores. In both cases, however, derivatisation was effected in 50mM phosphate buffer, pH 7.8, followed by transfer to 50mM phosphate buffer, pH 6.8, as quickly as possible after derivatisation to stabilise the maleimide group.

Example 7: Test results analysing the coupling of FMDV peptide and of HRV2 peptide to PV cores

Tests were carried out to analyse the PV cores with FMDV peptide attached, obtained in Examples 2 and 6, and with HRV2 peptide attached, obtained in Examples 3 and 6, as follows:

(a) Polyacrylamide gel electrophoresis. This showed that all of the core protein had been shifted to a higher molecular weight due to the addition of peptide.

(b) Western blotting. Following gel electrophoresis proteins were transferred to nitro cellulose and reacted with anticore or antipeptide antisera. The results showed that the higher molecular weight forms of the protein appearing after coupling reacted with both antisera as expected.

(c) ELISA.

(i) Assays were performed by a sandwich method in which anticore antiserum bound to the ELISA plate was used to trap derivatised core particles. The tests were developed following reaction with antipeptide antiserum. These showed that the derivatised particles had both core and peptide antigenicity.

(ii) Titration by ELISA of anti-HRV2 peptide antiserum against fusion core particles where the HRV2 peptide sequence was fused to the N-terminus of the core protein and against PV cores to which the HRV2 peptide was coupled gave similar results. As equal amounts of the two particles were used in the assay, the antigenic activity of the N-terminal fusion particles and the chemically coupled particles was substantially the same. The anti-HRV2 peptide antiserum was antiserum raised against the HRV2 peptide attached in Examples 3 and 6.

(d) Sucrose density gradients. This analysis showed that coupling of the FMDV peptide to cores resulted in particle aggregation. They pelleted to the bottom of the tube. PV cores coupled with HRV peptide sedimented to the same position as untreated cores and had both core and peptide immunogenicity by ELISA.

(e) Electron microscopy. PV cores coupled to HRV2 peptide have been examined and are regular looking particles. Electron microscopy of immune complexes formed between PV cores coupled to HRV2 peptide and anti-HRV2 peptide antiserum showed that the core particles were linked by antibody.

(f) Immunogenicity.

(i) FMDV peptide coupled to cores gave neutralizing activity with <20μg of coupled material (<2μg peptide).

(ii) The immunogenic activity of the HRV2 peptide N-terminal fusion core particles and the chemically coupled particles was compared in guinea pigs using 20 μg doses of each antigen. The antisera were tested at various times post-injection by ELISA against the HRV2 peptide. The results were:

| Day | Chemically coupled core | fusion core |
|---|---|---|
| 0 | <1 | <1 |
| 14 | 2.9 | 2.3 |
| 28 | 3.9 | 3.3 |
| 56 | 3.9 | 4.0 |
| boost | | |
| 63 | 4.1 | 3.9 |
| 70 | 4.1 | 3.9 |
| 84 | 4.2 | 3.9 |

The antigens were injected intramuscularly in incomplete Freunds adjuvant.

Example 8: Preparation of FMDV tandem repeat peptides

The peptides shown below were synthesised by the solid-phase method. More specifically, synthesis was carried out using an adaption of the Merrifield technique (Merrifield, JACS 85 2149-2154, 1963) described by Houghten (Houghten, Proc. Natl. Acad. Sci. USA 82 5131-5135, 1985). Each peptide has an additional non-natural cysteine residue at its C-terminus except for peptide 199 which has a C-terminal non-natural glycine residue.

Each peptide was synthesised on a p-methyl-benzhydrylamine divinylbenzene resin. The alpha-amino protecting group on each amino acid was t-butoxycarbonyl (Boc). Each coupling cycle was as follows:
1. Wash resin with dichloromethane - 10 minutes
2. Wash with 5% diisopropylethylamine in dichloromethane - 2 minutes x 3
3. Dichloromethane wash - 1 minute x 2
4. Couple t-butoxycarbonyl amino acid dichloromethane, 0.3M diisopropylcarbodiimide - 60 minutes
5. As 3
6. Deprotect with 50% trifluoroacetic acid in dichloromethane - 20 minutes
7. Dichloromethane wash - 1 minutes x 6
8. Return to 2.

When the coupling cycles were completed the peptide was cleaved off the resin using hydrogen fluoride for 1 hour with an anisole scavenger 10%. The peptide was thus obtained with a carboxy-terminal amide group. It was then ether washed, dried, dissolved in 15% acetic acid and lyophilized.

| VIRUS | PEPTIDE | REFERENCE NUMBER |
|---|---|---|

FMDV $O_1/O_1$    137-162-137-162-Cys
$O_1$        $O_1$

198

$(NRNAVPNLRGDLQVLAQKVARTLPTS)_{x2} C$

FMDV $O_1/O_1$    137-162-137-162-Gly
$O_1$        $O_1$

199

$(NRNAVPNLRGDLQVLAQKVARTLPTS)_{x2} G$

FMDV $O_1/O_1$    137-162-137-162-Cys
$O_1$        $O_1$

315

$(NRNAVPNLRGDLQVLAQKVARTLPTS)_{x2} C$

FMDV $A_{12}B/A_{12}B$    137-162-137-162-Cys
$A_{12}$        $A_{12}$

318

$(YSASGSGVRGDLGSLAPRVARQLPAS)_{x2} C$

FMDV $O_1/O_1/O_1$    145-150-145-150-145-160-Cys
$O_1$    $O_1$    $O_1$

112

RGDLQVRGDLQVRGDLQVLAQKVARTLPC

FMDV $O_1/O_1$    145-150-145-160-Cys
$O_1$        $O_1$

113

RGDLQVRGDLQVLAQKVARTLPC

## Example 9: Preparation of FMDV mixed serotype tandem repeat peptides

The peptides shown below were synthesised by the solid-phase method described in Example 8.

| VIRUS | PEPTIDE | REFERENCE NUMBER |
|---|---|---|
| FMDV $O_1/A_{12}B$ | 137–162–137–162–Cys $O_1$  $A_{12}B$ NRNAVPNLRGDLQVLAQKVARTLPTS– YSASGSGVRGDLGSLAPRVARQLPAS–C | 316 |
| FMDV $A_{12}B/O_1$ | 137–162–137–162–Cys $A_{12}B$  $O_1$ YSASGSGVRGDLGSLAPRVARQLPAS– NRNAVPNLRGDLQVLAQKVARTLPTS–C | 317 |

Example 10: Preparation of HBcAg provided at its N-terminus with an extension comprising HRV2 VP2 residues 156 to 170 (HRV2 core)

To construct chimaeric fusion particles coding for a specific N-terminal HRV2 VP2 epitope comprising VP2 residues 156 to 170, synthetic oligonucleotides with cohesive ends for EcoRI (5') and BamHI (3') respectively were prepared using an Applied Biosystems 381A DNA synthesiser. These oligonucleotides were ligated into EcoRI-BamHI digested pBc404 which had been purified from 1% low melting point agarose by standard methods (Francis and Clarke, Meth. Enzymology 178, 659-676, 1989). This DNA was then transformed into E. coli strain JM101, and recombinant plasmids were restriction mapped from small scale DNA preparations. The synthetic oligonucleotides, how they anneal together and the coding sequence of the N-terminal extension are:

1. AATTCAGTTAAAGCGGAAACGCGTTTG

2. AACCCAGATCTGCAACCGACCGAATGCCGG

3. GATCCCGGCATTCGGTCGGTTGCA

4. GATCTGGGTTCAAACGCGTTTCCGCTTTAACTG


AATTCAG TTAAAGCGGA AACGCGTTTG AACCCAGATC TGCAACCGAC

     GTC AATTTCGCCT TTGCGCAAAC TTGGGTCTAG ACGTTGGCTG

CGAATCCGC

GCTTACGGCC CTAG

                          30

ATG AAT TCA GTT AAA GCG GAA ACG CGT TTG AAC CCA GAT
 M   N   S   V   K   A   E   T   R   L   N   P   D

   LINKER            HRV2 VP2 EPITOPE
                               60

CTG CAA CCG ACC GAA TGC CGG GAT CC...core
 L   Q   P   T   E   C   R   D

Bacteria harbouring recombinant plasmids were grown overnight in L-Amp medium to high cell density and diluted with fresh L-broth (1:10) the following day. Expression was routinely induced by the immediate addition of IPTG (60 µg/ml final concentration) and the bacteria allowed to replicate for a further 6-8 hr at 37°C. Bacteria were then harvested and chimaeric core particles, with N-terminal peptide epitopes, purified and characterised as previously described (Clarke et al, Nature 330, 381-383, 1987; Francis and Clarke, 1989).

Example 11: Coupling of HBsAg to HRV2 cores

HRV2 cores from Example 10 were derivatised with an equimolar amount of SMCC in DMF. The amount of SMCC was one-twentieth the volume of DMF. Yeast-expressed HBsAg were derivatised at 200 µg/ml with SATA in equimolar ratios of DMF. The amount of SATA was one-twentieth the volume of DMF. After deacetylation of the SATA with hydroxylamine to produce free -SH groups, equal volumes of the two derivatised particles, each of 200 µg/ml, were mixed. The HRV2 cores with HBsAg attached were recovered.

**Claims**

**Claims for the following Contracting States : BE, CH, DE, DK, FR, GB, IT, LI, NL, SE**

1. Hepatitis B core antigen (HBcAg) particles which carry a polypeptide which presents an antigenic epitope, characterised in that the HBcAg particles are composed of HBcAg provided with a N-terminal extension incorporating a Lys residue within the first fourteen N-terminal amino acid residues of the extension and the said polypeptide is coupled to the particles via the side chain amino group of the Lys residue.

2. Particles according to claim 1, wherein the polypeptide comprises an antigenic epitope capable of raising neutralising antibodies.

3. Particles according to claim 2, wherein the epitope is an epitope of a virus, bacterium, or protozoan.

4. Particles according to any one of the preceding claims, wherein the polypeptide is up to 50 amino acid residues long.

5. Particles according to any one of the preceding claims, wherein the N-terminal extension is up to 60 amino acid residues long.

6. Particles according to claim 5, wherein the N-terminal extension is up to 40 amino acid residues long.

7. Particles according to claim 6, wherein the N-terminal extension is up to 20 amino acid residues long.

8. Particles according to any one of the preceding claims, wherein the N-terminal extension comprises residues 95 to 102 of the VP1 capsid protein of a type 1 poliovirus or residues 156 to 164 of the VP2 capsid protein of human rhinovirus (HRV) type 2 or equivalent amino acid residues of another HRV.

9. Particles according to any one of the preceding claims, wherein one Lys residue is present within the first twelve N-terminal amino acid residues of the extension.

10. Particles according to claim 9, wherein one Lys residue is present within the first five N-terminal amino acid residues of the extension.

11. Particles according to any one of the preceding claims, wherein two Lys residues are present within the first fourteen N-terminal amino acid residues of the extension.

12. Particles according to any one of the preceding claims, wherein the HBcAg provided with the N-terminal extension is carboxymethylated.

13. A process for the preparation of HBcAg particles which carry a polypeptide which presents an antigenic epitope, characterised in that the polypeptide is coupled to HBcAg particles, which are composed of HBcAg provided with a N-terminal extension incorporating an exposed Lys residue within the first fourteen N-terminal amino acid residues of the extension, via the side chain amino group of the Lys residue.

14. A process according to claim 13, in which the HBcAg provided with the N-terminal extension is carboxymethylated prior to coupling of the polypeptide.

15. A pharmaceutical composition comprising a pharmaceutically acceptable carrier or diluent and, as active principle, HBcAg particles as defined in any one of claims 1 to 12 or which have been prepared by a process as claimed in claim 13 or 14 which carry a polypeptide which presents an antigenic epitope.

**Claims for the following Contracting State : ES**

1. A process for the preparation of HBcAg particles which carry a polypeptide which presents an antigenic epitope, which process comprises the step of coupling the polypeptide to HBcAg particles, which are composed of HBcAg provided with a N-terminal extension incorporating an exposed Lys residue within the first fourteen N-terminal amino acid residues of the extension, via the side chain amino group of the Lys residue.

2. A process according to claim 1, in which the HBcAg provided with the N-terminal extension is carboxymethylated prior to coupling of the polypeptide.

3. A process according to claim 1 or 2, wherein the polypeptide comprises an antigenic epitope capable of raising neutralising antibodies.

4. A process according to claim 3, wherein the epitope is an epitope selected from an epitope of a virus, an epitope of a bacterium and an epitope of a protozoan.

5. A process according to any one of the preceding claims, wherein the polypeptide is up to 50 amino acid residues long.

6. A process according to any one of the preceding claims, wherein the N-terminal extension is up to 60 amino acid residues long.

7. A process according to claim 6, wherein the N-terminal extension is up to 40 amino acid residues long.

8. A process according to claim 7, wherein the N-terminal extension is up to 20 amino acid residues long.

9. A process according to any one of the preceding claims, wherein the N-terminal extension is selected from an extension which comprises residues 95 to 102 of the VP1 capsid protein of a type 1 poliovirus and an extension which comprises residues 156 to 164 of the VP2 capsid protein of human rhinovirus (HRV) type 2 or equivalent residues of another HRV.

10. A process according to any one of preceding claims, wherein one Lys residue is present within the first twelve N-terminal amino acid residues of the extension.

11. A process according to claim 10, wherein one Lys residue is present within the first five N-terminal amino acid residues of the extension.

12. A process according to any one of the preceding claims, wherein two Lys residues are present within the first fourteen N-terminal amino acid residues of the extension.

13. A process according to any one of the preceding claims, further comprising forming a pharmaceutical composition by formulating a pharmaceutically acceptable carrier or diluent with the said HBcAg particles carrying the polypeptide which presents an antigenic epitope which have been obtained.


**Patentansprüche**

**Patentansprüche für folgende Vertragsstaaten CH, DE, DK, FR, GB, IT, LI, NL, SE, BE**

1. Hepatitis B-Kernantigen (HBcAg)-Teilchen, die ein Polypeptid tragen, das ein antigenes Epitop aufweist, dadurch gekennzeichnet, daß die HBcAg-Teilchen zusammengesetzt sind aus HBcAg, das mit einer N-endständigen Erweiterung mit einem Lys-Rest innerhalb der ersten 14 N-endständigen Aminosäurereste der Erweiterung versehen ist, wobei das Polypeptid an die Teilchen über die Seitenketten-Aminogruppe des Lys-Restes gekuppelt ist.

2. Teilchen nach Anspruch 1, wobei das Polypeptid ein antigenes Epitop aufweist, das zur Bildung von neutralisierenden Antikörpern befähigt ist.

3. Teilchen nach Anspruch 2, wobei es sich beim Epitop um ein Epitop eines Virus, Bakteriums oder Protozoons handelt.

4. Teilchen nach einem der vorstehenden Ansprüche, wobei das Polypeptid eine Lange von bis zu 50 Aminosäureresten aufweist.

5. Teilchen nach einem der vorstehenden Ansprüche, wobei die N-endständige Erweiterung eine Lange von bis zu 60 Aminosäureresten aufweist.

6. Teilchen nach Anspruch 5, wobei die N-endständige Erweiterung eine Länge von bis zu 40 Aminosäureresten aufweist.

7. Teilchen nach Anspruch 6, wobei die N-endständige Erweiterung eine Länge von bis zu 20 Aminosäureresten aufweist.

8. Teilchen nach einem der vorstehenden Ansprüche, wobei die N-endständige Erweiterung die Reste 95-102 des VP1-Capsid-Proteins eines Typ 1-Poliovirus oder die Reste 155-164 des VP2-Capsid-Proteins von humanem Rhinovirus (HRV) Typ 2 oder äquivalente Aminosäurereste eines anderen HRV umfaßt.

9. Teilchen nach einem der vorstehenden Ansprüche, wobei 1 Lys-Rest innerhalb der ersten 12 N-endständigen Aminosäurereste der Erweiterung vorhanden ist.

10. Teilchen nach Anspruch 9, wobei ein Lys-Rest innerhalb der ersten 5 N-endständigen Aminosäurereste der Erweiterung vorhanden ist.

11. Teilchen nach einem der vorstehenden Ansprüche, wobei 2 Lys-Reste innerhalb der ersten 14 N-endständigen Aminosäurereste der Erweiterung vorhanden sind.

12. Teilchen nach einem der vorstehenden Ansprüche, wobei das HBcAg, das mit der N-endständigen Erweiterung versehen ist, carboxymethyliert ist.

13. Verfahren zur Herstellung von HBcAg-Teilchen, die ein Polypeptid tragen, das ein antigenes Epitop aufweist, dadurch gekennzeichnet, daß das Polypeptid an HBcAg-Teilchen, die aus HBcAG, das eine N-endständige Erweiterung mit einem freiliegenden Lys-Rest innerhalb der ersten 14 N-endständigen Aminosäurereste der Erweiterung aufweist, zusammengesetzt sind, über die Seitenkette-Aminogruppe des Lys-Restes gekuppelt ist.

14. Verfahren nach Anspruch 13, wobei das HBcAg der N-endständigen Erweiterung vor der Kupplung des Polypeptids carboxymethyliert wird.

15. Pharmazeutische Zusammensetzung, enthaltend einen pharmazeutisch verträglichen Trägerstoff oder Verdünnungsmittel und als Wirkstoff HBcAg-Teilchen, die der Definition in einem der Ansprüche 1-12 entsprechen oder die gemäß einem Verfahren nach Anspruch 13 oder 14 hergestellt worden sind, die ein Polypeptid mit einem antigenen Epitop tragen.

**Patentansprüche für folgenden Vertragsstaat : ES**

1. Verfahren zur Herstellung von HBcAg-Teilchen, die ein Polypeptid tragen, das ein antigenes Epitop aufweist, umfassend die Stufe der Kupplung des Polypeptids an die HBcAg-Teilchen, die aus HBcAg, das mit einer N-endständigen Erweiterung mit einem freiliegenden Lys-Rest innerhalb der ersten 14 N-endständigen Aminosäurereste der Erweiterung versehen ist, zusammengesetzt sind, über die Seitenketten-Aminogruppe des Lys-Restes.

2. Verfahren nach Anspruch 1, wobei das HBcAg mit der N-endständigen Erweiterung vor der Kupplung des Polypeptids carboxymethyliert worden ist.

3. Verfahren nach Anspruch 1 oder 2, wobei das Polypeptid ein antigenes Epitop aufweist, das zur Bildung von neutralisierenden Antikörpern befähigt ist.

4. Verfahren nach Anspruch 3, wobei es sich beim Epitop um ein Epitop eines Virus, Bakteriums oder Protozoons handelt.

5. Teilchen nach einem der vorstehenden Ansprüche, wobei das Polypeptid eine Länge von bis zu 50 Aminosäureresten aufweist.

6. Verfahren nach einem der vorstehenden Ansprüche, wobei die N-endständige Erweiterung eine Länge von bis zu 60 Aminosäureresten aufweist.

7. Verfahren nach Anspruch 6, wobei die N-endständige Erweiterung eine Länge von bis zu 40 Aminosäureresten aufweist.

8. Verfahren nach Anspruch 7, wobei die N-endständige Erweiterung eine Länge von bis zu 20 Aminosäureresten aufweist.

9. Verfahren nach einem der vorstehenden Ansprüche, wobei die N-endständige Erweiterung ausgewählt ist unter Erweiterungen, die die Reste 95-102 des VP1-Capsid-Proteins eines Typ 1-Poliovirus oder die Reste 156-164 des VP2-Capsid-Proteins von humanem Rhinovirus (HRV) Typ 2 oder äquivalente Reste eines anderen HRV umfassen.

10. Verfahren nach einem der vorstehenden Ansprüche, wobei 1 Lys-Rest innerhalb der ersten 12 N-endständigen Aminosäurereste der Erweiterung vorhanden ist.

11. Verfahren nach Anspruch 10, wobei ein Lys-Rest innerhalb der ersten 5 N-endständigen Aminosäurereste der Erweiterung vorhanden ist.

12. Verfahren nach einem der vorstehenden Ansprüche, wobei 2 Lys-Reste innerhalb der ersten 14 N-endständigen Aminosäurereste der Erweiterung vorhanden sind.

13. Verfahren nach einem der vorstehenden Ansprüche, ferner umfassend die Bildung einer pharmazeutischen Zusammensetzung durch Herrichten eines pharmazeutisch verträglichen Trägers oder Verdünnungsmittels mit den erhaltenen HBcAg-Teilchen, die das Polypeptid tragen, das ein antigenes Epitop auf-

weist.

## Revendications

### Revendications pour les Etats contractants suivants : BE, CH, DE, DK, FR, GB, IT, LI, NL, SE

1. Particules de l'antigène du coeur de l'hépatite B (HBcAg) qui portent un polypeptide qui présente un épitope antigénique, caractérisées en ce que les particules HBcAg sont composées de HBcAg muni d'une extension N-terminale incorporant un résidu lysine dans les quatorze premiers résidus acide aminé N-terminaux de l'extension et en ce que le polypeptide est couplé aux particules par le radical amino de chaîne latérale au résidu lysine.

2. Particules suivant la revendication 1, où le polypeptide comprend un épitope antigénique capable de produire des anticorps neutralisants.

3. Particules suivant la revendication 2, où l'épitope est un épitope de virus, de bactérie ou de protozoaire.

4. Particules suivant l'une quelconque des revendications précédentes, où le polypeptide a une longueur de jusqu'à 50 résidus acide aminé.

5. Particules suivant l'une quelconque des revendications précédentes, où l'extension N-terminale a une longueur de jusqu'à 60 résidus acide aminé.

6. Particules suivant la revendication 5, où l'extension N-terminale a une longueur de jusqu'à 40 résidus acide aminé.

7. Particules suivant la revendication 6, où l'extension N-terminale a une longueur de jusqu'à 20 résidus acide aminé.

8. Particules suivant l'une quelconque des revendications précédentes, où l'extension N-terminale comprend les résidus 95 à 102 de la protéine de capside VP1 d'un poliovirus de type 1 ou les résidus 156 à 164 de la protéine du capside VP2 du rhinovirus humain (HRV) de type 2 ou les résidus acide aminé équivalents d'un autre HRV.

9. Particules suivant l'une quelconque des revendications précédentes, où un résidu lysine est présent dans les douze premiers résidus acide aminé N-terminaux de l'extension.

10. Particules suivant la revendication 9, où un résidu lysine est présent dans les cinq premiers résidus acide aminé N-terminaux de l'extension.

11. Particules suivant l'une quelconque des revendications précédentes, où deux résidus lysine sont présents dans les quatorze premiers résidus acide aminé N-terminaux de l'extension.

12. Particules suivant l'une quelconque des revendications précédentes, où le HBcAg muni de l'extension N-terminale est carboxyméthylé.

13. Procédé pour la préparation de particules de HBcAg qui portent un polypeptide qui présente un épitope antigénique, caractérisé en ce que le polypeptide est couplé aux particules HBcAg, qui sont composées de HBcAg muni d'une extension N-terminale incorporant un résidu lysine exposé dans quatorze premiers résidus acide aminé N-terminaux de l'extension par le radical amino de chaîne latérale du résidu lysine.

14. Procédé suivant la revendication 13, dans lequel le HBcAg muni de l'extension N-terminale est carboxyméthylé avant le couplage avec le polypeptide.

15. Composition pharmaceutique comprenant un excipient ou diluant pharmaceutiquement acceptable et, comme principe actif, des particules HBcAg définies dans l'une quelconque des revendications 1 à 12 ou qui ont été préparées par un procédé suivant la revendication 13 ou 14 qui porte un polypeptide qui présente un épitope antigénique.

**Revendications pour l'Etat contractant suivant : ES**

1. Procédé de préparation de particules HBcAg qui portent un polypeptide qui présente un épitope antigénique, lequel procédé comprend l'étape de couplage du polypeptide aux particules HBcAg, qui sont composées d'HBcAg muni d'une extension N-terminale incorporant un résidu lysine exposé dans les quatorze premiers résidus acide aminé N-terminaux de l'extension, par le radical amino de la chaîne latérale du résidu lysine.

2. Procédé suivant la revendication 1, dans lequel le HBcAg muni de l'extension N-terminale est carboxyméthylé avant le couplage au polypeptide.

3. Procédé suivant la revendication 1 ou 2, dans lequel le polypeptide comprend un épitope antigénique capable de produire des anticorps neutralisants.

4. Procédé suivant la revendication 3, dans lequel l'épitope est un épitope choisi parmi un épitope de virus, un épitope de bactérie et un épitope de protozoaire.

5. Procédé suivant l'une quelconque des revendications précédentes, dans lequel le polypeptide a une longueur de jusqu'à 50 résidus acide aminé.

6. Procédé suivant l'une quelconque des revendications précédentes, dans lequel l'extension N-terminale a une longueur de jusqu'à 60 résidus acide aminé.

7. Procédé suivant la revendication 6, dans lequel l'extension N-terminale a une longueur de jusqu'à 40 résidus acide aminé.

8. Procédé suivant la revendication 7, dans lequel l'extension N-terminale a une longueur de jusqu'à 20 résidus acide aminé.

9. Procédé suivant l'une quelconque des revendications précédentes, dans lequel l'extension N-terminale est choisie parmi une extension qui comprend les résidus 95 à 102 de la protéine de capside VP1 d'un poliovirus de type 1 et une extension qui comprend les résidus 156 à 164 de la protéine de capside VP2 d'un rhinovirus humain (HRV) de type 2 ou des résidus équivalents d'un autre HRV.

10. Procédé suivant l'une quelconque des revendications précédentes, dans lequel un résidu lysine est présent dans les douze premiers résidus acide aminé N-terminaux de l'extension.

11. Procédé suivant la revendication 10, dans lequel un résidu lysine est présent dans les cinq premiers résidus acide aminé N-terminaux de l'extension.

12. Procédé suivant l'une quelconque des revendications précédentes, dans lequel deux résidus lysine sont présents dans les quatorze premiers résidus acide aminé N-terminaux de l'extension.

13. Procédé suivant l'une quelconque des revendications précédentes, comprenant, de plus, la formation d'une composition pharmaceutique par formulation d'un diluant ou excipient pharmaceutiquement acceptable avec les particules HBcAg portant le polypeptide qui présente un épitope antigénique qui ont été obtenues.

HBcAg

pBc404

B

E

P

tac

bla

ori

HBcAg

Tac promoter --ACACAGGAAACAGTT ATG AATTC-------GGATCCGCGCGCCCTTGGGTGGCTTTGGGG

SD

EcoRI

BamHI

HBcAg